(19) Europäisches Patentamt
European Patent Office
Office européen des brevets

(11) Veröffentlichungsnummer: **0 019 059**
**B1**

(12) # EUROPÄISCHE PATENTSCHRIFT

(45) Veröffentlichungstag der Patentschrift: **29.12.82**

(51) Int. Cl.³: **C 07 J 53/00** // C07J9/00

(21) Anmeldenummer: **80101504.1**

(22) Anmeldetag: **21.03.80**

(54) **Verfahren zur Herstellung von Cholestenderivaten und neue Zwischenprodukte in deren Herstellung.**

(30) Priorität: **18.05.79 CH 4676/79**

(43) Veröffentlichungstag der Anmeldung: **26.11.80 Patentblatt 80/24**

(45) Bekanntmachung des Hinweises auf die Patenterteilung: **29.12.82 Patentblatt 82/52**

(84) Benannte Vertragsstaaten: **AT BE CH DE FR GB IT NL**

(56) Entgegenhaltungen:
**EP-A-0 004 098**
**FR-A-2 301 537**
**FR-A-2 400 526**
**STEROIDS, Band 17, Nr. 6, Juni 1971, HOLDEN DAY, SAN FRANCISCO (US) J.E., HERZ et al.: »Steroidal triphenyl phosphonium salts, versatile intermediates for side chain modifications«, Seiten 649—652.**

(73) Patentinhaber: **F. HOFFMANN-LA ROCHE & CO. Aktiengesellschaft, CH-4002 Basel (CH)**

(72) Erfinder: **Barner, Richard, Dr., In Den Reben 97, D-4108 Witterswil (CH)**
Erfinder: **Hübscher, Josef, Spausel 1080, CH-5703 Seon (CH)**

(74) Vertreter: **Mahé, Jean et al, Postfach 601 Grenzacherstrasse 124, CH-4002 Basel (CH)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



BUNDESDRUCKEREI BERLIN

Verfahren zur Herstellung von Cholestenderivaten
und neue Zwischenprodukte in deren Herstellung

Die Erfindung betrifft ein Verfahren zur Herstellung von Cholestenderivaten, die als Zwischenprodukte bei der Herstellung von 25,26-Dihydroxycholecalciferol Verwendung finden können. Diese Cholestenderivate besitzen die allgemeine Formel

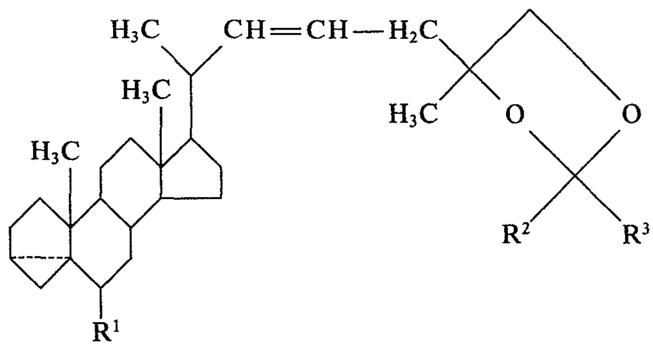

(I)

worin $R^1$ nieder-Alkoxy und $R^2$ und $R^3$ nieder-Alkyl oder $R^2$ und $R^3$ zusammen nieder-Alkylen sind.

Beispiele von niederen Alkylresten $R^2$ und $R^3$ sind Methyl, Äthyl und Propyl, wobei Methyl bevorzugt ist. Beispiele für durch $R^2$ und $R^3$ gemeinsam gebildete Alkylenreste sind Äthylen und Propylen. Beispiele von niederen Alkoxygruppen sind $C_{1-6}$-Alkoxyreste, insbesondere Methoxy.

Das erfindungsgemäße Verfahren ist dadurch gekennzeichnet, daß man eine Verbindung der Formel

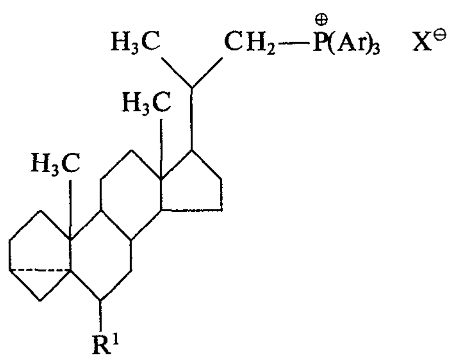

(II)

worin $R^1$ die obige Bedeutung hat, Ar einen Arylrest und $X^{\ominus}$ ein Anion darstellt, in einer Wittig-Reaktion mit einer Verbindung der Formel

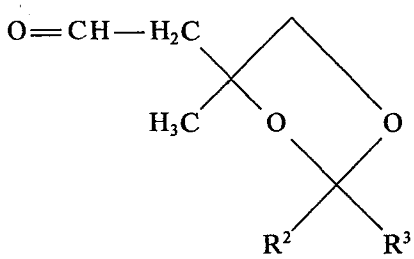

(III)

worin $R^2$ und $R^3$ die obige Bedeutung haben,
umsetzt.

Die in den FR-A-2 400 526 und 2 301 537 beschriebenen 25-Hydroxycholestenderivate werden ausgehend von einem Steroidaldehyd und einem der Seitenkette des herzustellenden Cholestens entsprechenden Ylid erhalten. Im Gegensatz hierzu werden die 25,26-Dihydroxy-cholestenderivate I durch Umsetzung des dem steroidalen Phosphoniumsalz II entsprechenden Ylids mit dem der Seitenkette des Cholestens I entsprechenden Aldehyd hergestellt.

Beispiele von Arylresten Ar sind gegebenenfalls durch nieder-Alkyl oder -Alkoxy substituiertes Phenyl, vorzugsweise Phenyl. Beispiele von Anionen $X^{\ominus}$ sind Halogenionen, vorzugsweise $I^{\ominus}$.

Die Reaktion kann unter den für Wittig-Reaktionen bekannten Bedingungen durchgeführt werden. Als Lösungsmittel kommen z. B. Äther, wie Tetrahydrofuran, Dioxan oder Diäthyläther; oder Kohlenwasserstoffe, wie Toluol, in Betracht; als Basen Butyllithium, Natriumhydrid, Natriumamid oder

Kalium-tert.-butylat.

Vorzugsweise nimmt man die Herstellung des Ylids bei niedrigen Temperaturen, z. B. bei −50° bis −80°, vor, um die Möglichkeit der Spaltung der i-Steroid-Gruppierung auszuschließen.

Die Erfindung betrifft weiterhin die neuen Verbindungen der Formel II.

Die Verbindungen der Formel II werden erfindungsgemäß dadurch hergestellt, daß man eine Verbindung der Formel

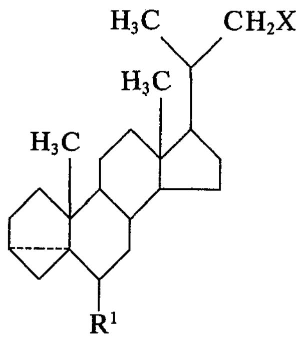

(IV)

worin X Halogen und $R^1$ nieder-Alkoxy ist,
mit einem Triarylphosphin in Gegenwart von Kaliumcarbonat in Acetonitrit bei etwa 100° C umsetzt.

Die Cholestenderivate der Formel I können durch Hydrierung der Doppelbindung in entsprechende Cholestanderivate umgewandelt werden, die nach Ketalspaltung und retro-i-Umlagerung 25,26-Dihydroxycholesterin liefern, das in bekannter Weise in 25,26-Dihydroxycholecalciferol übergeführt werden kann.

Die Hydrierung der Doppelbindung in einer Verbindung der Formel I kann katalytisch, z. B. in Gegenwart von Edelmetallen wie Platin, Palladium oder Rhodium, oder mit Nickelkatalysatoren durchgeführt werden.

Die retro-i-Umlagerung kann durch Behandlung mit einer Säure in einem geeigneten solvolytischen Medium bewerkstelligt werden. Als solvolytisches Medium kommen wäßrige Medien in Betracht, die ein mischbares Co-Solvens enthalten. Geeignete Co-Solventien sind ätherische Lösungsmittel, wie Tetrahydrofuran oder Dioxan, oder Ketone, wie Aceton und Methyläthylketon, oder Alkohole, wie Äthanol. Als Säuren kommen Mineralsäuren, wie Salzsäure, Bromwasserstoffsäure oder Schwefelsäure, oder organische Sulfonsäuren, wie Benzolsulfonsäure oder p-Toluolsulfonsäure, in Betracht. Bei Verwendung dieser Säuren wird gleichzeitig die Ketalgruppe hydrolysiert.

Das so erhaltene 25,26-Dihydroxy-cholesterin kann, z. B. wie in Steroids 24 (1974), Seite 463, beschrieben, in 25,26-Dihydroxy-cholecalciferol übergeführt werden.

Die folgenden Beispiele erläutern die Erfindung.

## Beispiel 1

9,35 g (20S)-21-Jod-6β-methoxy-20-methyl-3α,5-cyclo-5α-pregnan, 6,64 g Triphenylphosphin und 6,6 g pulverisiertes Kaliumcarbonat werden in 17 ml Acetonitril 12 Stunden bei 100° (Badtemperatur) kräftig gerührt. Aus der Suspension wurden 10 ml Acetonitril abdestilliert, und es wurde weitere 6 Stunden bei 100° gerührt. Das Reaktionsgemisch wurde mit 300 ml Aceton verrührt, filtriert, der Rückstand mit Aceton gewaschen und das Filtrat eingeengt. Das erhaltene viskose Öl wurde mit Äther verrührt, wobei Kristallisation eintrat; die Kristalle wurden abfiltriert und mit Äther gewaschen. Zur Reinigung wurden das Phosphoniumsalz (13,25 g) in 175 ml Aceton gelöst und mit 500 ml Äther wieder gefällt, filtriert, mit Äther gewaschen und im Hochvakuum getrocknet. Man erhielt 11,7 g (79,5%) [(20S)-6β-Methoxy-20-methyl-3α,5-cyclo-5α-pregnan-21-yl]-triphenylphosphoniumjodid vom Smp. 230−231°.

## Beispiel 2

Zu einer Suspension von 3,59 g [(20S)-6β-Methoxy-20-methyl-3α,5-cyclo-5α-pregnan-21-yl]-triphenylphosphoniumjodid in 100 ml trockenem Tetrahydrofuran wurden unter Stickstoffatmosphäre bei −78° 3,75 ml einer 2 M-Lösung von Butyllithium in Hexan zugetropft. Zur vollständigen Bildung des Ylids wurde noch 1/2 Stunde bei −78° und 1/2 Stunde bei −25° nachgerührt, wobei eine tieforangerote Lösung erhalten wurde. Bei unveränderter Temperatur wurden unter Rühren 790 mg (±)-2,2,4-Trimethyl-1,3-dioxolan-4-acetaldehyd (86% gemäß Gaschromatographie) zugetropft, und das Reaktionsgemisch wurde anschließend noch 30 Minuten bei −20°, 1 Stunde bei 0° und 1 Stunde bei Raumtemperatur gerührt. Dann wurde das Reaktionsgemisch mit 200 ml Eiswasser und 200 ml

Äther aufgenommen, die Ätherphase im Scheidetrichter abgetrennt und die wäßrige Phase noch dreimal mit je 200 ml Äther nachextrahiert. Die vereinigten Ätherphasen wurden über Magnesiumsulfat getrocknet und am Rotationsverdampfer eingeengt. Der Rückstand wurde an Kieselgel mit Toluol-Essigester (2 : 1) chromatographiert, wobei 1,48 g (73%) 6β-Methoxy-24 – [2,2,4-trimethyl-1,3-dioxolan-4-yl]-22-dehydro-3α,5-cyclo-5α-cholan erhalten wurden. Diese Verbindung kann wie folgt in 25,26-Dihydroxy-cholesterin umgewandelt werden:

Eine Lösung von 1,29 g 6β-Methoxy-24 -- [2,2,4-trimethyl-1,3-dioxolan-4-yl]-22-dehydro-3α,5-cyclo-5α-cholan in 50 ml Essigester werden über 0,8 g Pt-Kohle 10% bei Raumtemperatur hydriert (1 Stunde; $H_2$-Aufnahme 79 ml). Nach Abfiltrieren des Katalysators und Eindampfen des Filtrats wird 6β-Methoxy-24-[2,2,4-trimethyl-1,3-dioxolan-4-yl]-3α,5-cyclo-5α-cholan erhalten.

7,8 g 6β-Methoxy-24-[2,2,4-trimethyl-1,3-dioxolan-4-yl]-3α,5-cyclo-5α-cholan wurden in 200 ml Dioxan gelöst und nach Zusatz von 0,3 g p-Toluolsulfonsäure und 60 ml Wasser 3 Stunden zum Rückfluß erhitzt. Danach wurde das Reaktionsgemisch 12 Stunden bei Raumtemperatur gerührt und mit 200 ml Äther versetzt. Die wäßrige Phase wurde mit zweimal 50 ml Äther gewaschen, und die vereinigten organischen Phasen über Magnesiumsulfat getrocknet und eingedampft. Der Rückstand wurde in Äther suspendiert, die Suspension filtriert und der Rückstand mit Äther gewaschen. Nach Trocknen des Rückstandes erhielt man 3,6 g 25,26-Dihydroxy-cholesterin, Schmelzpunkt 173 – 176°, $[\alpha]_D$ – 35,8° (c = 0,4% in Methanol).

**Patentansprüche für die Vertragsstaaten: BE, CH, DE, FR, GB, IT, NL**

1. Verfahren zur Herstellung von Cholestenderivaten der Formel

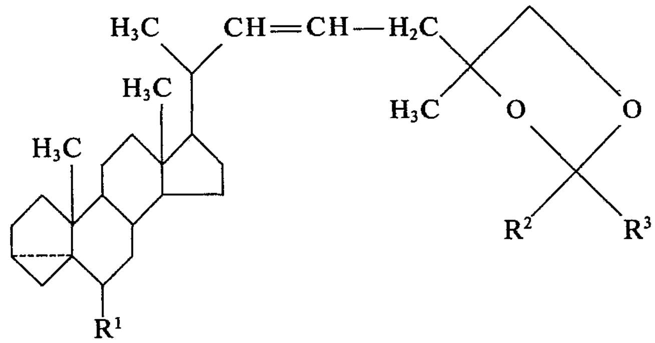

(I)

worin $R^1$ nieder-Alkoxy und $R^2$ und $R^3$ nieder-Alkyl oder $R^2$ und $R^3$ zusammen nieder-Alkylen sind, dadurch gekennzeichnet, daß man eine Verbindung der Formel

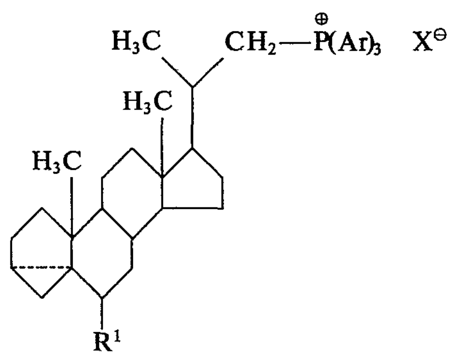

(II)

worin $R^1$ die obige Bedeutung hat, Ar einen Arylrest und $X^\ominus$ ein Anion darstellt, in einer Wittig-Reaktion mit einer Verbindung der Formel

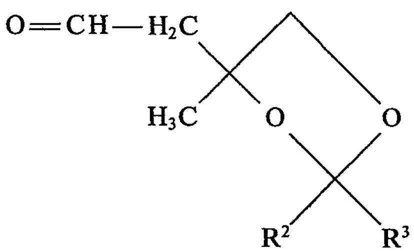

(III)

worin $R^2$ und $R^3$ die obige Bedeutung haben, umsetzt.

2. Verfahren nach Anspruch 1, dadurch gekennzeichnet, daß man die Verbindung der Formel II durch Umsetzung einer Verbindung der Formel

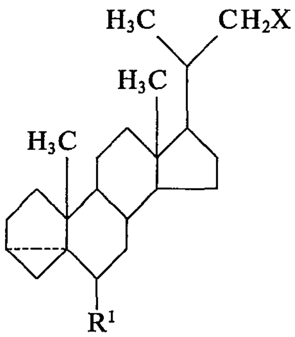

(IV)

worin X Halogen und $R^1$ nieder-Alkoxy ist, mit einem Triarylphosphin in Gegenwart von Kaliumcarbonat in Acetonitril bei etwa 100° C herstellt.

3. Verfahren nach Anspruch 1, dadurch gekennzeichnet, daß man [(20S)-6β-Methoxy-20-methyl-3α,5-cyclo-5α-pregnen-21-yl]-triphenylphosphonium-jodid bei niedriger Temperatur mit Butyllithium in das Ylid überführt und dieses mit 2,2,4-Trimethyl-1,3-dioxolan-4-acetaldehyd umsetzt.

4. Verfahren nach Anspruch 2, dadurch gekennzeichnet, daß man (20S)-21-Jod-6β-pregnan mit Triphenylphosphin umsetzt.

5. Verbindungen der Formel

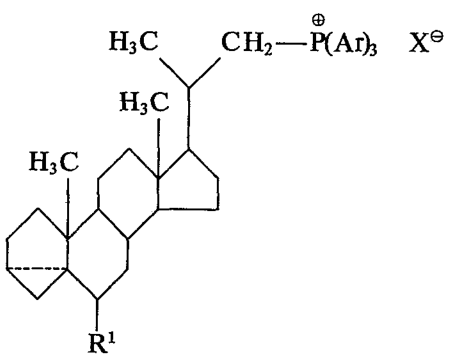

(II)

worin $R^1$ nieder-Alkoxy, Ar ein Arylrest und $X^\ominus$ ein Anion ist.

6. [(20S)-6β-Methoxy-20-methyl-3α,5-cyclo-5α-pregnan-21-yl]-triphenylphosphoniumjodid.

**Patentansprüche für den Vertragsstaat: AT**

1. Verfahren zur Herstellung von Cholestenderivaten der Formel

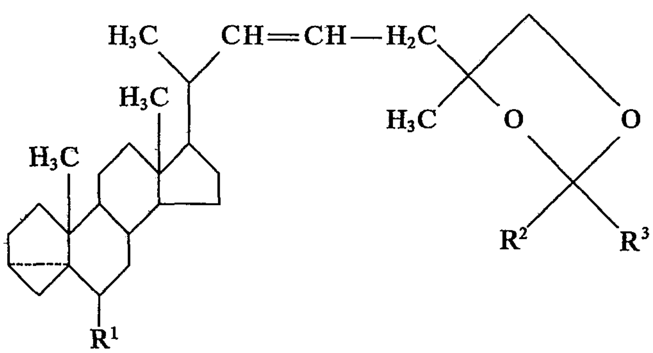

(I)

worin $R^1$ nieder-Alkoxy und $R^2$ und $R^3$ nieder-Alkyl oder $R^2$ und $R^3$ zusammen nieder-Alkylen sind, dadurch gekennzeichnet, daß man eine Verbindung der Formel

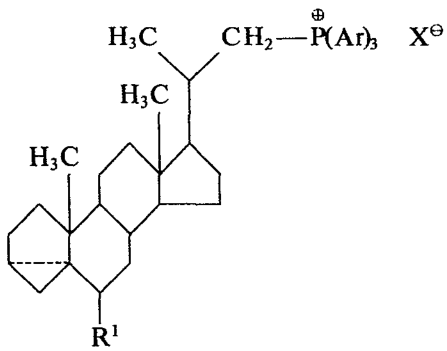

(II)

worin $R^1$ die obige Bedeutung hat, Ar einen Arylrest und $X^{\ominus}$ ein Anion darstellt, in einer Wittig-Reaktion mit einer Verbindung der Formel

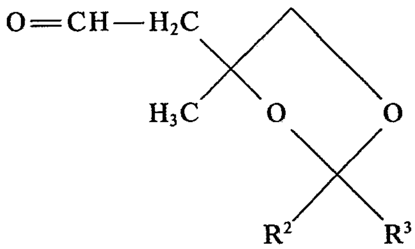

(III)

worin $R^2$ und $R^3$ die obige Bedeutung haben, umsetzt.

2. Verfahren nach Anspruch 1, dadurch gekennzeichnet, daß man die Verbindung der Formel II durch Umsetzung einer Verbindung der Formel

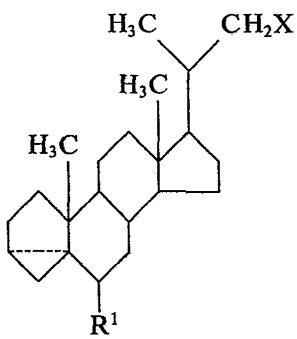

(IV)

worin X Halogen und $R^1$ nieder-Alkoxy ist,
mit einem Triarylphosphin in Gegenwart von Kaliumcarbonat in Acetonitrit bei etwa 100° C herstellt.

3. Verfahren nach Anspruch 1, dadurch gekennzeichnet, daß man [(20S)-6β-Methoxy-20-methyl-3α,5-cyclo-5α-pregnen-21-yl]-triphenylphosphonium-jodid bei niedriger Temperatur mit Butyllithium in das Ylid überführt und dieses mit 2,2,4-Trimethyl-1,3-dioxolan-4-acetaldehyd umsetzt.

4. Verfahren nach Anspruch 2, dadurch gekennzeichnet, daß man (20S)-21-Jod-6β-pregnan mit Triphenylphosphin umsetzt.

**Claims for the contracting states: BE, CH, DE, FR, GB, IT, NL**

1. Process for the manufacture of cholestene derivatives of the formula

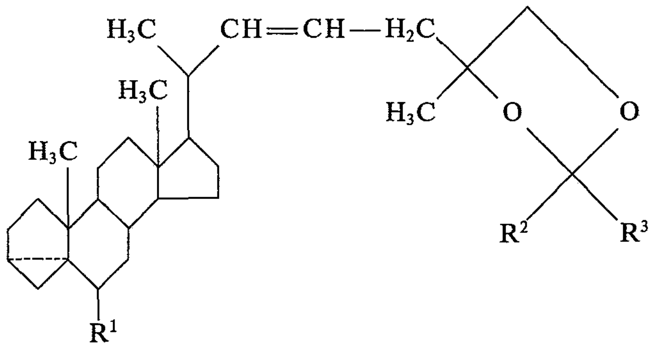

(I)

wherein $R^1$ is lower-alkoxy and $R^2$ and $R^3$ are lower-alkyl or $R^2$ and $R^3$ together are lower-alkylene, characterized by reacting a compound of the formula

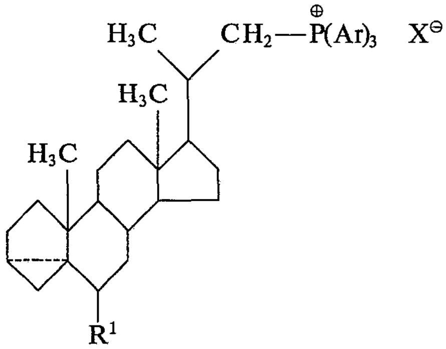

(II)

wherein $R^1$ has the above significance, Ar represents an aryl residue and $X^{\ominus}$ represents an anion, in a Wittig reaction with a compound of the formula

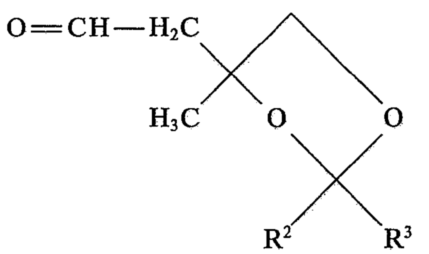

(III)

wherein $R^2$ and $R^3$ have the above significance.

2. Process according to claim 1, characterized in that the compound of formula II is prepared by reacting a compound of the formula

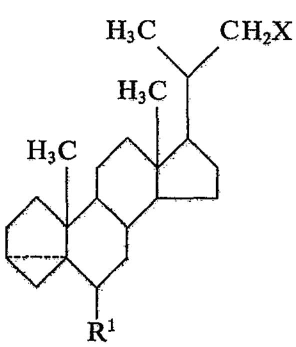

(IV)

wherein X is halogen and $R^1$ is lower-alkoxy, with a triarylphosphine in the presence of potassium carbonate in acetonitrile at about 100° C.

3. Process according to claim 1, characterized in that [(20S)-6β-methoxy-20-methyl-3α,5-cyclo-5α-pregnen-21-yl]-triphenylphosphonium iodide is converted at low temperature with butyl lithium into the ylid and this is reacted with 2,2,4-trimethyl-1,3-dioxolan-4-acetaldehyde.

4. Process according to claim 2, characterized in that (20S)-21-iodo-6β-pregnane is reacted with triphenylphosphine.

5. Compounds of the formula

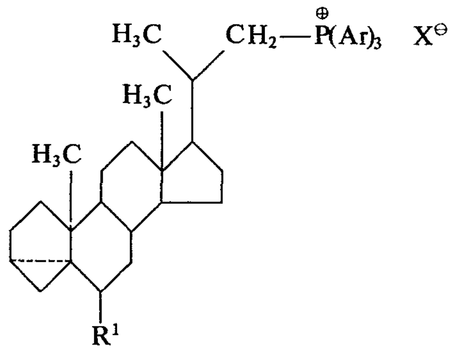

(II)

wherein $R^1$ is lower-alkoxy, Ar is an aryl residue and $X^\ominus$ is an anion.

6. [(20S)-6β-Methoxy-20-methyl-3α,5-cyclo-5α-pregnan-21-yl]-triphenylphosphonium iodide.

**Claims for the contracting state: AT**

1. Process for the manufacture of cholestene derivatives of the formula

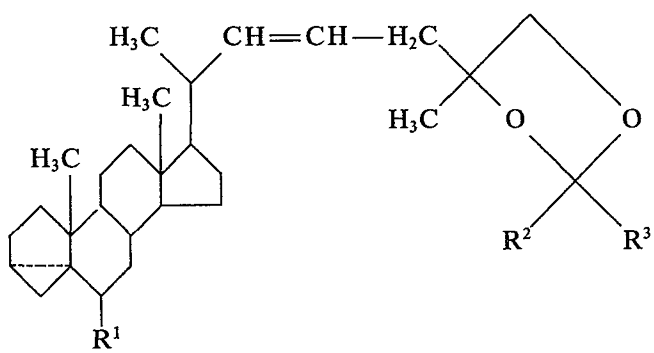

(I)

wherein $R^1$ is lower-alkoxy and $R^2$ and $R^3$ are lower-alkyl or $R^2$ and $R^3$ together are lower-alkylene, characterized by reacting a compound of the formula

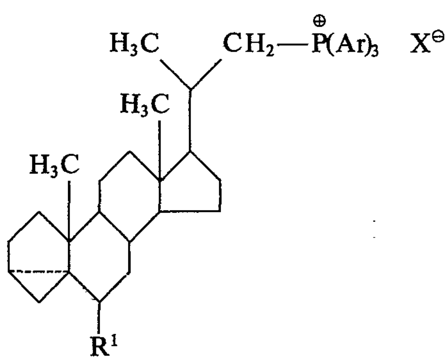

(II)

wherein $R^1$ has the above significance, Ar represents an aryl residue and $X^\ominus$ represents an anion, in a Wittig reaction with a compound of the formula

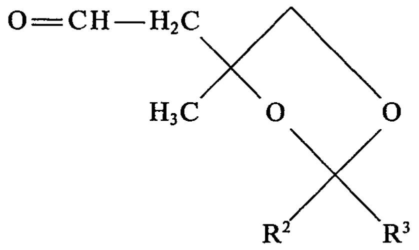

(III)

wherein $R^2$ and $R^3$ have the above significance.

2. Process according to claim 1, characterized in that the compound of formula II is prepared by reacting a compound of the formula

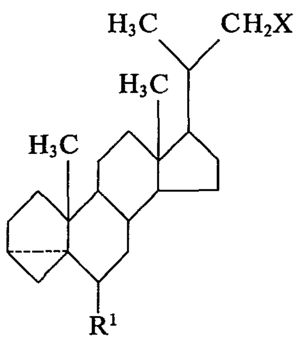

(IV)

wherein X is halogen and $R^1$ is lower-alkoxy,
with a triarylphosphine in the presence of potassium carbonate in acetonitrile at about 100° C.

3. Process according to claim 1, characterized in that [(20S)-6β-methoxy-20-methyl-3α,5-cyclo-5α-pregnen-21-yl]-triphenylphosphonium iodide is converted at low temperature with butyl lithium into the ylid and this is reacted with 2,2,4-trimethyl-1,3-dioxolan-4-acetaldehyde.

4. Process according to claim 2, characterized in that (20S)-21-iodo-6β-pregnane is reacted with triphenylphosphine.

**Revendications pour les Etats contractants: BE, CH, DE, FR, GB, IT, NL**

1. Procédé de préparation de dérivés du cholestène de formule:

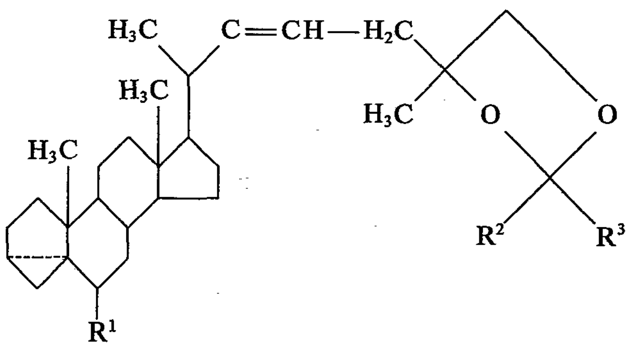

(I)

dans laquelle $R^1$ représente un groupe alcoxy inférieur et $R^2$ et $R^3$ des groupes alkyles inférieurs ou bien $R^2$ et $R^3$ forment ensemble un groupe alkylène inférieur,
caractérisé en ce que l'on fait réagir un composé de formule:

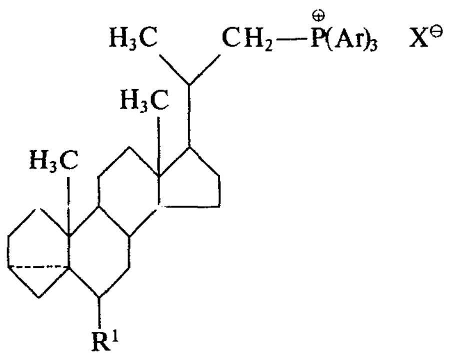

(II)

dans laquelle $R^1$ a la signification indiquée ci-dessus, Ar représente un reste aryle et $X^{\ominus}$ représente un anion,
dans une réaction de Wittig, avec un composé de formule:

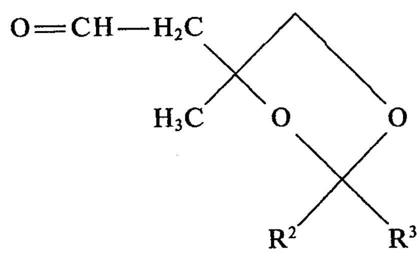

(III)

dans laquelle $R^2$ et $R^3$ ont les significations indiquées ci-dessus.

2. Procédé selon la revendication 1, caractérisé en ce que l'on prépare le composé de formule (II) en faisant réagir un composé de formule:

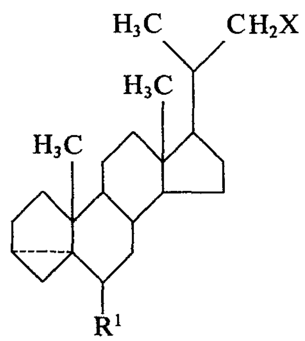

(IV)

dans laquelle X représente un halogène et $R^1$ un groupe alcoxy inférieur,
avec une triarylphosphine en présence de carbonate de potassium dans l'acétonitrile à une température d'environ 100° C.

3. Procédé selon la revendication 1, caractérisé en ce que l'on convertit l'iodure de [(20S)-6β-méthoxy-20-méthyl-3α,5-cyclo-5α-pregnène-21-yl]-triphénylphosphonium, à basse température, à l'aide du butyllithium, en l'ylide qu'on fait réagir avec le 2,2,4-triméthyl-1,3-dioxolanne-4-acétaldéhyde.

4. Procédé selon la revendication 2, caractérisé en ce que l'on fait réagir le (20S)-21-iodo-6β-pregnane avec la triphénylphosphine.

5. Composés de formule:

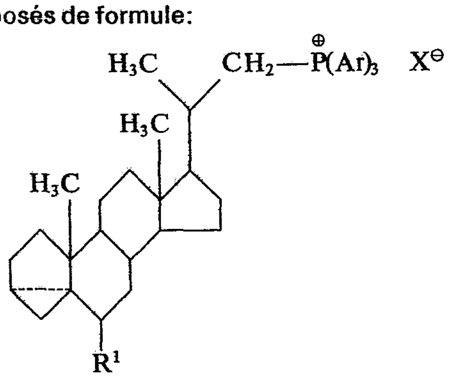

(II)

dans laquelle $R^1$ représente un groupe alcoxy inférieur, Ar un reste aryle et $X^{\ominus}$ un anion.

6. L'iodure de [(20S)-6β-méthoxy-20-méthyl-3α,5-cyclo-5α-pregnane-21-yl]-triphénylphosphonium.

**Revendications pour l'Etat contractant: AT**

1. Procédé de préparation de dérivés du cholestène de formule:

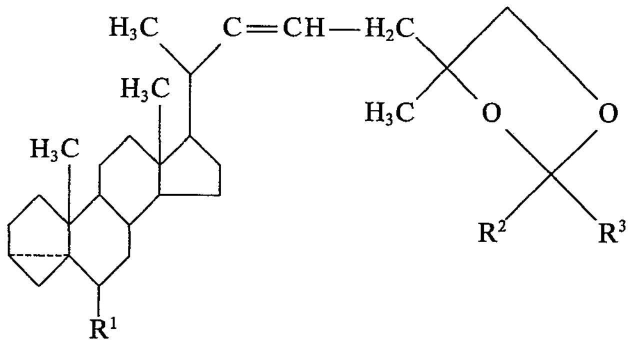

(I)

dans laquelle $R^1$ représente un groupe alcoxy inférieur et $R^2$ et $R^3$ des groupes alkyles inférieurs ou bien $R^2$ et $R^3$ forment ensemble un groupe alkylène inférieur, caractérisé en ce que l'on fait réagir un composé de formule:

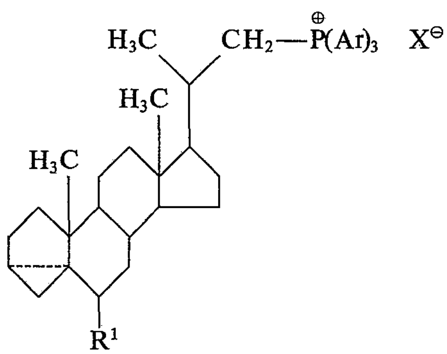

(II)

dans laquelle $R^1$ a la signification indiquée ci-dessus, Ar représente un reste aryle et $X^{\ominus}$ représente un anion, dans une réaction de Wittig, avec un composé de formule:

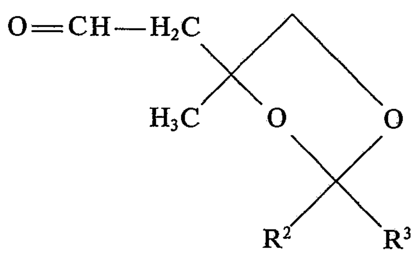

(III)

dans laquelle $R^2$ et $R^3$ ont les significations indiquées ci-dessus.

2. Procédé selon la revendication 1, caractérisé en ce que l'on prépare le composé de formule (II) en faisant réagir un composé de formule:

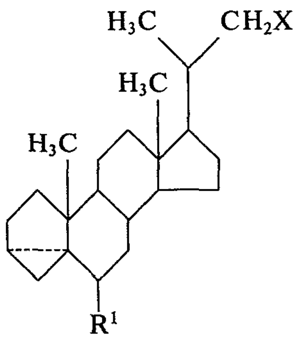

(IV)

dans laquelle X représente un halogène et R[1] un groupe alcoxy inférieur, avec une triarylphosphine en présence de carbonate de potassium dans l'acétonitrile à une température d'environ 100° C.

3. Procédé selon la revendication 1, caractérisé en ce que l'on convertit l'iodure de [(20S)-6β-méthoxy-20-méthyl-3α,5-cyclo-5α-pregnène-21-yl]-triphénylphosphonium, à basse température, à l'aide du butyllithium, en l'ylide qu'on fait réagir avec le 2,2,4-triméthyl-1,3-dioxolanne-4-acétaldéhyde.

4. Procédé selon la revendication 2, caractérisé en ce que l'on fait réagir le (20S)-21-iodo-6β-pregnane avec la triphénylphosphine.